# FASCICULE DE BREVET EUROPEEN

(11) **EP 2 635 315 B1**
(45) Date de publication et mention de la délivrance du brevet: **06.06.2018**
(21) Numéro de dépôt: 11791226.1
(22) Date de dépôt: 04.11.2011
(51) Int. Cl.: A61L 27/44, A61L 27/46, A61L 27/48, B29L 31/00, A61L 27/54, A61L 27/58, A61F 2/30, A61K 6/033, A61K 6/087, B29B 7/00, B29C 71/00, B29B 7/82, B29B 7/90, B29B 9/06, B29K 71/00, B29K 105/06, B29C 45/00, A61L 27/18, A61K 6/00, A61F 2/28

(54) **PROCEDE D'OBTENTION D'UN MATERIAU DE COMBLEMENT OSSEUX ET MATERIAU OBTENU PAR UN TEL PROCEDE**
VERFAHREN ZUR HERSTELLUNG EINES KNOCHENFÜLLERMATERIALS UND NACH DIESEM VERFAHREN HERGESTELLTES MATERIAL
METHOD FOR PREPARING A BONE FILLER MATERIAL AND MATERIAL PREPARED BY SUCH A METHOD

(30) Priorité: 04.11.2010 FR 1004325
(43) Date de publication de la demande: 11.09.2013
(73) Titulaire: Ethical Medical Implants SAS, 33700 Merignac (FR)
(72) Inventeur: COUGOULIC, Jean-Pierre, 44380 Pornichet (FR)
(74) Mandataire: Ipside
(86) Numéro de dépôt international: PCT/EP2011/069486
(87) Numéro de publication internationale: WO 2012/059599

(56) Documents cités:
- FR-A1- 2 722 694
- US-A1- 2003 170 378
- US-B1- 7 517 539

## Description

L'invention concerne un procédé pour l'obtention d'un matériau de comblement osseux et un matériau obtenu par un tel procédé. L'invention est plus particulièrement, mais non exclusivement, destinée à la réalisation d'implants endo-osseux, notamment dentaires, ou à la réalisation de prothèses osseuses pour des applications médicales ou vétérinaires. L'invention vise à l'obtention d'un matériau synthétique ostéomimétique apte à réaliser une greffe avec un tissu osseux, ledit matériau pouvant être mis sous la forme d'une pièce, ou implant, de forme prédéfinie, de propriétés mécaniques suffisantes pour suppléer mécaniquement ledit tissu, dès son implantation dans celui-ci, au moins jusqu'à la réalisation et la consolidation de la greffe, en visant également une ostéointégration rapide.

Le phosphate de calcium est le constituant structurel essentiel d'un tissu osseux. Celui-ci se trouve dans ledit tissu sous la forme d'une apatite phosphocalcique polysubstituée de composition :

Ca_{8,3}(PO)_{4,3}(HPO₄, CO₃)_{1,7}(OH₂CO₃)_{0,3}

Cette forme est totalement non stoechiométrique, caractérisée par un ratio Ca/P différent de 1,67, valeur de ce ratio correspond aux apatites stoechiométriques notamment l'hydroxyapatite de calcium (Ca₅ (PO₄)₃OH) ou le dihydroxyapatite de calcium (Ca₁₀(PO₄)₆H₂O).

Selon l'art antérieur, un principe de greffe dans un tissu osseux peut être obtenu par l'utilisation de composés de phosphate de calcium résorbables, c'est-à-dire faiblement cristallins et non stoechiométriques avec un rapport Ca/P sensiblement différent de 1,67, lesquels composés de calcium doivent, en outre, être nanostructurés en termes de porosité. Ces caractéristiques favorisent l'action combinée des ostéoclastes et des ostéoblastes conduisant à l'ostéointégration de l'implant dans le tissu.

Le document US 7 517 539 décrit un ciment basé sur l'utilisation de phosphate de calcium hydraté qui peut être injecté dans un tissu osseux sous la forme d'une pâte et durcir par une réaction exothermique afin de former une structure présentant les caractéristiques requises de porosité nanostructurée et de non-stoechiométrie. Néanmoins si ce ciment permet d'atteindre une partie des objectifs visés par l'invention celui-ci ne permet pas la réalisation d'une pièce apte à suppléer mécaniquement le tissu osseux dès son implantation dans celui-ci.

Le document FR 2 722 694 décrit un matériau et un procédé d'obtention d'une pièce constituée de ce matériau selon un procédé de moulage par injection. Selon cet art antérieur, ledit matériau consiste en un composite formé d'une matrice polymère thermoplastique biocompatible chargée, notamment, avec des poudres réalisant un apport de phosphate de calcium sous la forme de dihydroxyapatite stoechiométrique et de phosphate tricalcique (Ca₃(PO₄)₂). Ce matériau présente des caractéristiques mécaniques élevées, notamment en regard de la résistance aux chocs, et module d'élasticité proche de celui de l'os. La présence de composés de phosphate de calcium, favorise l'intégration dudit matériau dans le tissu. Néanmoins, ces composés n'étant pas résorbables, et la surface de l'implant en contact avec les tissus n'étant pas nanostructurée, ni même poreuse, par le fait du procédé de moulage par injection, cette ostéointégration est lente.

Le document DE100 55 465 décrit un matériau composite consistant en une matrice polymère thermoplastique chargée de dihydroxyapatite sous forme stoechiométrique, et un procédé d'obtention d'une pièce constituée de ce matériau par frittage laser sélectif. Ce procédé permet d'obtenir une pièce de porosité nanostructurée mais dans son volume, de sorte que cette porosité se répercute négativement sur les propriétés mécaniques de la pièce. Par ailleurs, l'hydroxyapatite n'étant pas sous une forme résorbable, l'ostéointégration de la pièce dans le tissu est également lente.

Le document FR 2 848 856 décrit un matériau et le procédé d'obtention en moulage par injection d'une pièce constituée en ce matériau. Ce document décrit en outre un traitement de décapage surfacique de la pièce moulée permettant l'obtention d'une surface poreuse nanostructurée en surface de la pièce. Le matériau moulé comprend une matrice constituée d'un polymère thermoplastique chargée par des composés réalisant un apport de calcium sous la forme de phosphate tricalcique, de monétite (CaHPO₄), de dihydroxyapatite et d'hydroxyapatite, ces apatites étant sous leur forme stoechiométrique. Après un traitement de surface, les composés contenant du calcium émergeant de la surface de la pièce sont partiellement résorbables, et, combinés à la porosité nanostructurée de ladite surface, accélèrent l'ostéointégration de la pièce en comparaison des arts antérieurs exposés ci-avant.
contenant du calcium émergeant de la surface de la pièce sont partiellement résorbables, et, combinés à la porosité nanostructurée de ladite surface, accélèrent l'ostéointégration de la pièce en comparaison des arts antérieurs exposés ci-avant.

La présente invention constitue un perfectionnement majeur de ce dernier art antérieur et vise à accélérer encore l'ostéointégration de l'implant constitué du matériau objet de l'invention. À cette fin, l'invention concerne un procédé pour l'obtention d'un matériau pour la constitution d'un implant endo-osseux à usage médical ou vétérinaire, ostéomimétique selon la revendication 1

Ainsi, le procédé objet de l'invention permet de combiner les caractéristiques mécaniques de la pièce moulée et d'utiliser avantageusement le cycle thermodynamique associé à ces conditions de moulage par injection, pour créer une apatite de calcium résorbable.

L'invention concerne également le matériau, selon la revendication 6, obtenu par le procédé de la revendication 1.

Un tel matériau peut être mis en forme par des techniques d'usinage et de soudage pour réaliser tout ou partie_de pièces destinées à être implantées dans un tissu osseux, et renferme les composés calciques résorbables facilitant son ostéointégration dans ledit tissu, lesquels composés peuvent être révélés en surface par des opérations d'usinage ou de décapage surfacique.

L'invention concerne également un implant endo-osseux, selon la revendication 7, constitué du matériau selon l'invention.

Ainsi en comparaison des implants obtenus par les procédés connus de l'art antérieur, l'implant objet de l'invention comprend, notamment en surface, une concentration plus importante de composés calciques résorbables, ce qui accélère son ostéointégration. Le coeur compact de l'implant lui confère une grande résilience mécanique et permet notamment de former tout ou partie de celui-ci sous la forme de zones élastiques déformables pour faciliter son accrochage mécanique primaire dans le tissu osseux.

L'invention peut être mise en oeuvre selon les modes de réalisation avantageux exposés ci-après, lesquels peuvent être considérés individuellement ou selon toute combinaison techniquement opérante.

Avantageusement, le polymère thermoplastique est constitué de polyétheréthercétone (PEEK). Ce polymère thermoplastique est en effet particulièrement biocompatible et présente un module élastique proche de celui de l'os ce qui minimise les incompatibilités de déformation élastique à l'interface entre l'implant et le tissu d'implantation.

Avantageusement, le procédé objet de l'invention comprend après l'étape i) une étape consistant à :
iii.incorporer dans le mélange d'un oxyde métallique biocompatibles et bioactif.

De tels composés participent notamment à la radio opacité de l'implant ainsi qu'à la cicatrisation.

Avantageusement, l'oxyde métallique incorporé est du dioxyde de titane (TiO2) sous la forme anatase. Ainsi, le dioxyde de titane sous sa forme anatase participe à la cicatrisation du site d'intervention chirurgical par sa fonction d'activation ostéoclastique.

Avantageusement, le procédé objet de l'invention comprend entre les étapes i) et ii) une étape consistant à :
d.incorporer au mélange de l'adénosine monophosphate

Ainsi, la présence d'adénosine monophosphate favorise les échanges transmembranaires ainsi que la régulation des principes de la phosphorylation dans le cytosol.

Selon un mode de réalisation particulier, le procédé objet de l'invention comprend entre les étapes i) et ii) un étape consistant à :
v. incorporer au mélange un pourcentage pondéral inférieur à 2 % d'hydroxyapatite non-stoechiométrique.

Ainsi la teneur d'hydroxyapatite non-stoechiométrique peut être légèrement augmentée sans augmenter la proportion de charges dans le matériau, c'est-à-dire sans fragiliser celui-ci.

Avantageusement la teneur en hydroxyapatite non-stoechiométrique du matériau objet de l'invention est supérieure à 2 % en poids.

Cette teneur élevée en hydroxyapatite non-stoechiométrique permet d'accélérer l'ostéointégration du matériau.

Avantageusement, l'épaisseur de la couche de surface poreuse de l'implant objet de l'invention est comprise entre 400 nm et 2000 nm. Cette épaisseur de couche de surface est suffisante pour assurer une pénétration de la greffe et assurer un accrochage fort de l'implant dans le tissu, sans affaiblir mécaniquement l'implant même dans ses parties très fines, et sans créer de contrainte d'incompatibilité de déformation entre ladite couche et le corps de l'implant, une fois la greffe réalisée.

L'invention est exposée ci-après selon ses modes de réalisation préférés, nullement limitatifs, et en référence aux figures 1 à 3, dans les quelles :
- la figure 1, est une vue de principe en coupe d'un implant selon un exemple de réalisation implanté dans un tissu osseux, avec figure 1A une vue de détail en coupe de la partie superficielle de l'implant et figure 1B une photomicrographie de la surface en microscopie électronique montrant la colonisation cellulaire de ladite surface ;
- la figure 2 représente un organigramme du procédé objet de l'invention ;
- et la figure 3 montre un spectre de diffraction des rayons X réalisé sur une pastille de matériau obtenu selon un exemple de réalisation du procédé objet de l'invention.

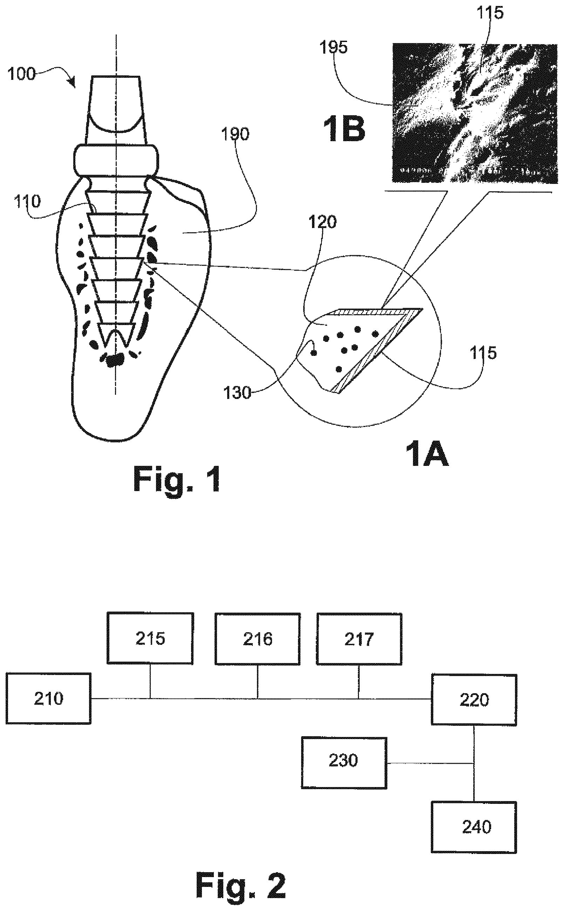

Figure 1, selon un exemple de réalisation non limitatif, l'invention concerne un implant endo-osseux (100), par exemple de type dentaire, apte à être implanté dans un maxillaire (190). Ledit implant (100) se présente avantageusement sous la forme d'une pièce moulée comprenant des reliefs (110) plus ou moins souples aptes à réaliser, par serrage latéral dans le puit d'implantation, l'accrochage mécanique primaire dudit implant. Cette capacité est obtenue par la souplesse et la résilience du polymère constituant la matrice de l'implant (100).

Figure 1A, selon une vue de détail en coupe, le matériau constituant l'implant comprend une matrice polymère (120) chargée par des particules (130) céramiques, notamment constituant un apport de calcium. La couche de surface (115) de l'implant est poreuse et nanostructurée.

Figure 1B, l'implant objet de l'invention réalise une greffe par la colonisation, en moins de 30 jours, de la couche de surface (115) de l'implant par les cellules (195) du tissu osseux. Cet implant (100), compact à coeur, et comprenant une couche de surface poreuse (115) nanostructurée laquelle contient des composés calciques (130) résorbables, est dit ostéomimétique, car d'une part, son module élastique, influencé essentiellement par le choix du polymère constituant la matrice (120), est proche du module élastique de l'os, et d'autre part, la couche poreuse (115) de surface favorise l'activité des ostéoclastes et des ostéoblastes du tissu receveur pour obtenir une ostéointégration rapide (figure 1B) de l'implant (100) dans ledit tissu receveur (190). En comparaison de l'art antérieur, l'ostéointégration est accélérée par la présence dans l'implant, et plus particulièrement dans la couche de surface (115), d'une proportion accrue, supérieur à 2% en poids, d'apatites non-stoechiométriques, résorbables et se rapprochant de l'hydroxyapatite constituant le substrat structural du tissu osseux. Ladite couche de surface (115) est d'une épaisseur comprise en 400 nm (10⁻⁹ m) et 2000 nm selon la nature des charges et le mode de décapage surfacique utilisé.

Figure 2, ces caractéristiques remarquables sont obtenues par la mise en oeuvre du procédé objet de l'invention. Selon un exemple de réalisation de ce procédé, celui-ci comprend une première étape (210), de préparation, consistant à mélanger de façon homogène un polymère thermoplastique biocompatible réalisant une structure de type matrice avec du phosphate tricalcique de type béta : le β TCP Ca₃(PO₄)₂. Ce phosphate tricalcique est choisi dans un grade dit « extra pur » sous forme poudreuse avec des grains de taille voisine de 200 µm (10⁻⁶ m). Ce grade de β TCP est, par exemple, commercialisé par la société VWR international Haasrode Researchpark Zone 3 GELDENAAKSEBAAN 464 B- 3001 LEUVEN Belgique.

Au cours d'une étape (220) d'injection, ledit mélange subit une opération de moulage sur une presse à injecter à vis. Cette étape d'injection est réalisée à une température au moins égale à la température de fusion du polymère constituant la matrice et préférentiellement entre 300 °C et 400 °C selon la nature du polymère mis en oeuvre. Le cycle thermodynamique de pression et de température subi par le mélange produit la transformation du phosphate tricalcique en cristaux résorbables et en molécules d'apatite de calcium non-stoechiométrique, donc résorbables.

La transformation lors de l'opération de moulage s'effectue selon la réaction :

4Ca (PO₄)₂+ 4(H₂0) = > 3((Ca₃(PO₄)₂)(OH)₂Ca + 2HPO₄ + ½O₂

3((Ca₃(PO₄)₂)OH₂)Ca correspond sensiblement à l'hydroxyapatite de l'os. Cette apatite est totalement non stoechiométrique, donc résorbable, ce qui permet de conférer au matériau des propriétés d'intégration de type greffe. Les pressions typiquement atteintes lors de l'opération d'injection/extrusion sont comprise entre 70 MPa et 140 MPa.

Les caractéristiques physico-chimiques des charges biologiques de type céramiques apatitiques non-stoechiométriques sont essentielles à leur activité biologique. Résorbables, elles conditionnent leur solubilité et donc leurs interactions avec le milieu biologique, à la base de leurs principales propriétés biologiques.

Cette apatite non-stoechiométrique résorbable similaire à celle de l'os favorise les échanges transmembranaires des ions calcium ainsi que les réactions transformatives des adénosines phosphates dans le cytosol.

La réaction ci-dessus s'effectue de façon partielle et le matériau final moulé contient aussi du phosphate tricalcique de type beta, résiduel. Ce phosphate tricalcique résiduel est également résorbable et offre avantageusement une fonction cicatrisante.

Le polymère thermoplastique constituant la matrice est avantageusement choisi parmi les polyéther éthercétone, les polyéther cétone, les polyethercétoneéthercétone, les polysulfone, les polytétrafluoro-éthylène, les polyaramide, les polyéther bloc amides ou les polyimide, sans que cette liste ne soit exhaustive. De préférence le polymère thermoplastique est un polyétheréthercétone (PEEK).

Le procédé objet de l'invention comprend optionnellement une étape (215) consistant à ajouter au mélange obtenu à l'étape précédente, des composés céramiques métalliques. Ainsi, plusieurs oxydes métalliques biocompatibles et bioactifs choisis parmi les céramiques des genres dioxyde de titane (TiO₂) sous forme anatase, dioxyde de zirconium (ZrO₂), ou oxyde d'aluminium (Al₂O₃) mais aussi du sulfate de baryum. Ces oxydes métalliques jouent un rôle de catalyseur dans la réaction chimique, de plus, leur masse molaire élevée permet de renforcer la radio-opacité du polymère thermoplastique constituant la matrice. Lesdits oxydes métalliques permettent aussi de réguler les concentrations ioniques des milieux biologiques rencontrés.

Plus particulièrement, le dioxyde de titane (TiO₂) sous forme anatase permet un nettoyage du site d'intervention de la chirurgie, favorisant ainsi une cicatrisation plus rapide, par sa fonction d'activation ostéoclastique.

Une autre étape optionnelle (216) du procédé objet de l'invention, consiste à réaliser une addition d'adénosine monophosphate lequel favorise les échanges transmembranaires ainsi que la régulation des principes de la phosphorylation dans le cytosol. Composant les sous unités des acides nucléiques en formant des nucléotides, il intègre donc les acides nucléiques et sert également à transporter l'énergie chimique dans leurs liaisons acide-anhydride facilement hydrolysées, mais peut aussi se lier à d'autres groupements pour former des enzymes qui sont des messagers spécifiques dans les cellules.

Associés aux ions calcium labiles contenus dans la formule apatitique non-stoechiométrique obtenue par l'injection/extrusion, il potentialise les processus de cicatrisation et donc d'acceptation biologique des pièces réalisées.

Enfin, selon une autre étape optionnelle du procédé objet de l'invention, celui-ci peut comprendre une addition (217) d'une petite proportion, inférieure à 2%, d'une apatite de calcium non-stoechiométrique au mélange obtenu précédemment.

Pour conserver un matériau moulable ayant la tenue et la résistance voulue, le polymère thermoplastique représente au moins 65 % en poids du matériau final. D'autre part, pour apporter suffisamment d'éléments chimiques destinés à favoriser l'intégration biologique, les composants complémentaires (acide phosphorique, phosphate tricalcique de type béta, apatite de calcium non stoechiométrique et oxyde métallique (TiO₂) associés à des composés calciques résorbables, représentent avantageusement entre 10 et 35 % en poids du matériau final

A l'issue de l'opération de moulage (220) la pièce obtenue peut subir des opérations optionnelles (230) de parachèvement tel que de l'usinage par enlèvement de matière afin de lui conférer la forme macroscopique désirée.

Finalement, au cours d'une dernière étape (240) la pièce subit une opération de décapage de surface visant à créer la couche superficielle poreuse. La stérilisation de la pièce est avantageusement réalisée au cours de cette opération de décapage. Un exemple de traitement de décapage conduisant à la formation d'une couche poreuse nanostructurée favorable à l'ostéointégration est décrit dans le document FR 2 848 856.

### Exemples :

Des mélanges de base sont réalisés à partir de polyéther éthercétone (PEEK), de phosphate tricalcique (Ca₃(PO₄)₂) de type béta, de dioxyde de titane (TiO₂) sous forme anatase et d'hydroxyapatite non stoechiométrique.

Un autre mélange de base est réalisé à partir de polyéther éthercétone (PEEK), de phosphate tricalcique (Ca₃(PO₄)₂) sous forme béta et de dioxyde de titane (TiO₂) de type anatase.

Le PEEK se présente sous la forme d'une poudre ou de granulés d'environ 100 µm, distribués par exemple par la Société EVONIK : Evonik Degussa GmbH Paul-Baumann-Str. 145772 Marl Germany

La référence de PEEK utilisée est dénommée VESTAKEEP® en poudre fine ou du PEEK 450P ceci notamment pour sa présentation en terme de granulométrie et pour sa pureté, afin d'optimiser la biocompatibilité du mélange avec les autres constituants.

Si la dihydroxyapatite de calcium est intégrée au mélange, elle l'est toujours sous une forme non-stoechiométrique. Ladite hydroxyapatite se présente sous la formed'une poudre blanche.

Le dioxyde de titane, sous forme anatase, se présente sous la forme d'une poudre dont les grains sont de taille supérieure à 200 µm. Elle est, par exemple, distribuée par VWR international Haasrode ResearchparkZone 3 GELDENAAKSEBAAN 464 B-3001 LEUVEN Belgique.

### a) Proportions

Les composés ci-dessus sont présents dans les proportions suivantes :

### Mélange 1 (10 % de charges)

- PEEK : 90 % en poids
- βTCP : 5 % en poids
- TiO₂ anatase : 4 % en poids
- Hydroxyapatite non-stoechiométrique : 1% en poids

### Mélange 2 (20 % de charges)

- PEEK : 80 % en poids
- βTCP : 9 % en poids
- TiO₂ : anatase 9 % en poids
- Hydroxyapatite non-stoéchiométrique : 2% en poids

### Mélange 3 (30 % de charges)

- PEEK :70 % en poids
- βTCP 14 % en poids
- TiO₂ : anatase 14 % en poids
- HYDROXYAPATITE 2 % en poids

### Mélange 4 (35 % de charges)

- PEEK :65 % en poids
- βTCP 16,5 % en poids
- TiO₂ :anatase 16,5 % en poids
- Hydroxyapatite non stoechiométrique : 2 % en poids

### Mélange 5

- PEEK : 80 % en poids
- βTCP 10 % en poids
- TiO₂ anatase : 10 % en poids

### b) Malaxage

Les constituants de chaque mélange sont placés dans un mélangeur à turbine jusqu'à obtention d'une parfaite homogénéisation.

### c) Séchage

Chaque mélange homogène obtenu est séché dans une étuve à circulation d'air pendant 3 heures à 150 °C.

### d) Extrusion- granulation

Cette opération permet d'obtenir dans une filière un filament ou un tube qui peut être coupé en morceaux de longueur différentes afin d'obtenir des granulés ou des tuyaux simples ou multiples de toutes les dimensions.

### e) Moulage

L'opération de moulage est réalisée sur presse à injecter de type FANUC électrique 30 tonnes,

Les conditions de préparation du matériel et les conditions de moulage du mélange correspondent à celles utilisées pour le PEEK pur.

Le PEEK étant un thermoplastique semi-cristallin, il est nécessaire de chauffer le moule, afin d'éviter la formation d'un voile de surface en phase amorphe. La thermorégulation du moule est assurée par un système de chauffage permettant de le maintenir à une température de l'ordre de 160°C.

Le moulage est réalisé à une température comprise entre 340 °C et 400 °C et à une pression d'injection comprise entre 70 et 140 MPa.

Le moule peut être conformé en fonction de la pièce à obtenir, par exemple pour la réalisation d'une prothèse osseuse, notamment pour des applications orthopédiques ou dentaires. Un bloc de matière peut également être moulé lequel est ensuite découpé ou usiné selon la forme désirée, pour un comblement osseux ou un implant, type cage intersomatique pour la colonne vertébrale par exemple. Selon les applications envisagées, des filaments peuvent être extrudés qui, après tricotage ou tissage, sont utilisés à titre de membrane de gainage, de protection ou de soutien ou bien des feuilles d'épaisseur variable, éventuellement percées de trous ou de micro trous, afin de réaliser des membranes.

### Résultats

### test d'analyse chimique

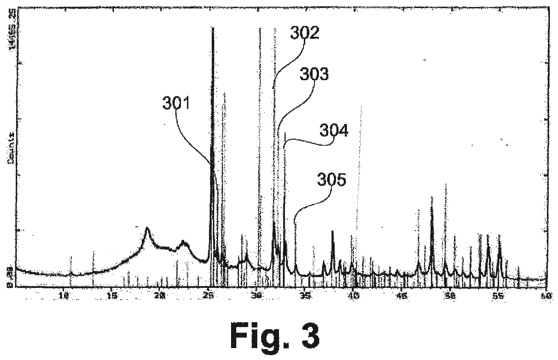

Figure 3, le spectre de diffraction X du mélange 5, c'est-à-dire à base de PEEK (80 %), phosphate tricalcique (10 %) de type béta et dioxyde de titane (10 %) sous forme anatase, réalisé sur une pastille après l'opération de moulage révèle, par la présence des pics (301,302, 303, 304, 305), la présence d'hydroxyapatite de calcium non-stoechiométrique dans le matériau moulé, du même type que la principale composante minérale de l'os humain.

Une biopsie est réalisée sur un implant, constitué d'un matériau correspondant au mélange 1, après implantation pendant 30 jours.

L'analyse histologique ne décrit aucune zone inflammatoire et surtout des prolongements cytoplasmiques ainsi que des cellules insérées dans la couche superficielle du matériau.

L'analyse par XPS décrit la présence d'azote dans le matériau à plus de 900 nanomètres. L'azote est liée au matériau par des liaisons covalentes. Il faut rappeler que le matériau ne contient absolument pas d'azote avant implantation. L'azote provient donc des cellules au contact avec le matériau.

Par ailleurs l'azote est liée à des radicaux contenant de l'hydrogène en réalisant des séquences aminées liées au matériau implanté. Cette analyse permet de conclure à un principe de greffe, induite, entre les cellules osseuses et le matériau.

La description ci-avant et les exemples de réalisation montrent que l'invention atteint des objectifs visés, en particulier, elle permet d'obtenir un matériau ostéomimétique à ostéointégration rapide.

## Revendications

1. Procédé pour l'obtention d'un matériau pour la constitution d'un implant endo-osseux à usage médical ou vétérinaire, ostéomimétique, **caractérisé en ce qu'**il comprend les étapes consistant à :
i. obtenir (210) un mélange homogène consistant en un polymère thermoplastique biocompatible et du phosphate tricalcique sous la forme béta (β TCP) ;
iv. incorporer (216) au mélange de l'adénosine monophosphate
ii. faire subir audit mélange une opération de moulage (220) par injection ou extrusion sur une machine a vis, a une température d'injection au moins égale à la température de fusion dudit polymère et une pression comprise entre 70 MPa et 140 MPa de sorte à transformer ledit phosphate tricalcique en une apatite de calcium3(Ca3(PO4)2)(OH)2Ca non stoechiométrique similaire a celle de l'os

2. Procédé selon la revendication 1, dans lequel le polymère thermoplastique est constitué de polyétheréthercétone (PEEK).

3. Procédé selon la revendication 1, comprenant entre les étapes i) et ii) un étape consistant à :
v. incorporer (217) au mélange un pourcentage pondéral inférieur à 2 % d'hydroxyapatite non stoechiométrique

4. Procédé selon la revendication 1, comprenant après l'étape i) une étape consistant à :
iii. incorporer (215) dans le mélange un oxyde métallique biocompatible et bioactif

5. Procédé selon la revendication 4, dans lequel l'oxyde métallique incorporé est du dioxyde de titane (TiO₂) sous la forme anatase.

6. Matériau obtenu par le procédé selon la revendication 1 comprenant :
a. 65 à 90 % de polymère thermoplastique
b. 10 à 35 % de composants complémentaires, au moins sous forme d'hydroxyapatite de calcium non stoechiométrique associée à des composés calciques résorbables
c. de l'adénosine monophosphate dans lequel la teneur en hydroxyapatite non-stoechiométrique est supérieure à 2 % en poids.

7. Implant endo-osseux (100) moulé, constitué du matériau selon la revendication 6 **caractérisé en ce qu'**il comprend :
x. un coeur compact (120)
y. une couche de surface poreuse (115) nanostructurée comprenant des composés calciques (130) résorbables, obtenue par décapage de la surface de l'implant moulé

8. Implant selon la revendication 7, dans lequel la teneur en hydroxyapatite non-stoechiométrique est supérieure à 2 % en poids.

9. Implant selon la revendication 7, dans lequel l'épaisseur de la couche poreuse (115) de surface est comprise entre 400 nm et 2000 nm.

## Patentansprüche

1. Verfahren zum Erhalten eines Materials für die Bildung eines osteomimetischen enossalen Implantats zur medizinischen oder veterinärmedizinischen Verwendung, **dadurch gekennzeichnet, dass** es die folgenden Schritte umfasst:
i. Erhalten (210) einer homogenen Mischung, die aus einem thermoplastischen biokompatiblen Polymer und aus Tricalciumphosphat in der Beta-Form (β-TCP) besteht,
iv. Einbringen (216) von Adenosinmonophosphat in die Mischung,
ii. Unterziehen der Mischung einer Formungsprozedur (220) durch Spritzgießen oder Extrusion auf einer Schneckenmaschine, bei einer Einspritztemperatur, die zumindest gleich der Schmelztemperatur des Polymers ist, und bei einem Druck im Bereich zwischen 70 MPa und 140 MPa, so dass das Tricalciumphosphat in einen nicht-stöchiometrischen Apatit von Calcium3 (Ca₃(PO₄)₂)(OH)₂Ca, ähnlich jenem von Knochen, umgewandelt wird.

2. Verfahren gemäß Anspruch 1, **dadurch gekennzeichnet, dass** das thermoplastische Polymer aus Polyetheretherketon (PEEK) gebildet ist.

3. Verfahren gemäß Anspruch 1, das zwischen den Schritten i) und i)) einen Schritt umfasst, bestehend aus:
v. Einbringen (217) von nicht-stöchiometrischem Hydroxyapatit bei einem Gewichtsprozentsatz unter 2% in die Mischung.

4. Verfahren gemäß Anspruch 1, das nach dem Schritt i) einen Schritt umfasst, bestehend aus:
iii. Einbringen (215) eines biokompatiblen und biowirksamen Metalloxids in die Mischung.

5. Verfahren gemäß Anspruch 4, wobei es sich bei dem eingebrachten Metalloxid um Titandioxid (TiO₂) in der Anatas-Form handelt.

6. Material, erhalten durch das Verfahren gemäß Anspruch 1, umfassend:
a. 65 bis 90% thermoplastisches Polymer,
b. wenigstens 10 bis 35% zusätzliche Bestandteile in der Form von nicht-stöchiometrischem Calcium-Hydroxyapatit, assoziiert mit resorbierbaren Calciumverbindungen,
c. Adenosinmonophosphat, wobei der Gehalt an nicht-stöchiometrischem Hydroxyapatit höher als 2 Gew.-% ist.

7. Geformtes enossales Implantat (100), das aus dem Material gemäß Anspruch 6 gebildet ist, **dadurch gekennzeichnet, dass** es umfasst:
x. einen kompakten Kern (120),
y. eine nanostrukturierte poröse Oberflächenschicht (115), umfassend resorbierbare Calciumverbindungen (130), erhalten durch Abbeizen der Oberfläche des geformten Implantats.

8. Implantat gemäß Anspruch 7, wobei der Gehalt an nicht-stöchiometrischem Hydroxyapatit höher als 2 Gew.-% ist.

9. Implantat gemäß Anspruch 7, wobei die Dicke der porösen Oberflächenschicht (115) zwischen 400 nm und 2000 nm beträgt.

## Claims

1. A method for obtaining a material for the constitution of an osteomimetic endo-osseous implant of medical or veterinary use, **characterized in that** it comprises the steps consisting in:
i. obtaining (210) a homogeneous mixture consisting in a biocompatible thermoplastic polymer and tricalcium phosphate in the beta form (β TCP),
iv. incorporating (216) adenosine monophosphate into the mixture
ii. subjecting that mixture to an injection or extrusion molding operation (220) by a screw injection machine, at an injection temperature at least equal to the melting temperature of the said polymer and a pressure of between 70 MPa and 140 MPa so as to transform said tricalcium phosphate into a non-stoichiometric calcium apatite 3(Ca₃(PO₄)₂)(OH)₂Ca similar to that of bone.

2. The method according to claim 1, wherein the thermoplastic polymer consists of polyether etherketone (PEEK).

3. The method according to claim 1, comprising between steps i) an ii) a step consisting in:
v. incorporating (217) into the mixture a weight percentage lower than 2% of non-stoichiometric hydroxyapatite.

4. The method according to claim 1, comprising after step i) a step consisting in:
iii. incorporating (215) into the mixture a biocompatible and bioactive metal oxide.

5. The method according to claim 4, wherein the incorporated metallic oxide is titanium dioxide (TiO₂) in the anatase form

6. A material obtained by the method according to claim 1, comprising:
a. 65 to 90% of a thermoplastic polymer;
b. 10 to 35% of complementary compounds, at least in the form of non-stoichiometric calcium hydroxyapatite associated with resorptive calcic compounds.
c. adenosine monophosphate
wherein its non-stoichiometric hydroxyapatite content is higher than 2% in weight.

7. An endo-osseous implant (100) made up of the material according to claim 6, **characterized in that** it comprises:
x. a compact core (120) ;
y. a nanostructured porous surface layer (115) comprising resorptive calcic compounds (130) obtained by etching the surface of the molded implant

8. The implant according to claim 7, wherein its non-stoichiometric hydroxyapatite content is higher than 2% in weight.

9. The implant according to claim 7, wherein the thickness of the porous surface layer (115) lies between 400 nm and 2000 nm.
